# EUROPEAN PATENT SPECIFICATION

(11) **EP 0 754 051 B1**
(45) Date of publication and mention of the grant of the patent: **27.08.2003**
(21) Application number: 95942270.0
(22) Date of filing: 27.12.1995
(51) Int. Cl.: A61K 38/18, A61P 3/06

(54) **THERAPEUTIC AGENT FOR DISORDER OF LIPID METABOLISM**
Therapeutische Mittel für Störung des Fettstoffwechsels
AGENT THERAPEUTIQUE DESTINE A TRAITER DES TROUBLES DU METABOLISME DES LIPIDES

(30) Priority: 27.12.1994 JP 33788494
(43) Date of publication of application: 22.01.1997
(73) Proprietor: DAIICHI PHARMACEUTICAL CO., LTD., Chuo-ku, Tokyo 103-8234 (JP)
(72) Inventor: SHIOTA, Akira, Shimotsuga-gun, Tochigi 329-43 (JP); FUJISE, Nobuaki, Ishibashi Heights 201, Shimotsuga-gun, Tochigi 329-055 (JP); MASUNAGA, Hiroaki, Shimotsuga-gun, Tochigi 321-02 (JP); HIGASHIO, Kanji, Kawagoe-shi, Saitama 350 (JP)
(74) Representative: Boeters, Hans Dietrich, Dr.
(86) International application number: JP9502707
(87) International publication number: WO96020004

(56) References cited:
- EP-A- 0 462 277
- EP-A- 0 588 477

## Description

### Field of the Invention

The present invention relates to the manufacture of a therapeutic agent for treatment of disorder of lipid metabolism comprising TCF-II as an effective ingredient. The therapeutic agent of the present invention is useful for treatment of disorder of lipid metabolism such as hypercholesterolemia, hypolipidemia, hypo-high-density-lipoproteinemia etc., which is induced by diabetes, endocrinological diseases, such as thyroid dysfunction and adrenal dysfunction, chronic metabolism disorder, chronicrenal failure including nephrosis, uremia, cancer, dyscrasia, adversed effects of antitumor agent, gastrointestinal(including hepatic and pancreatic) impairment and so on.

### Background of the Invention

More than 60% of the cause of Japanese death is adult disease, such as hypertension, arteriosclerosis, diabetes and so on. Generally, adult disease is caused mainly by deterioration of endogenous factor, such as endocrinological function and/or lipid metabolism function, whose controlling mechanism is not yet known in many aspects. Category of therapeutic agent for treatment of hyperlipidemia in terms of action mechanism is classified as absorption inhibition of cholesterol or choleic acid, inhibition of cholesterol synthesis and action on very low density protein metabolism (VLDL). Hyperlipidemia accompanied with decrease of high density protein(HDL)-cholesterol is a significant risk factor of arteriosclerotic diseases comprising ischemic heart disease, hypertension, ischemic encephalopathy and so on. It has been found that pharmacological effect of a therapeutic agent for treatment of hyperlipidemia is not only decreasing serum lipid, but also improving lowered HDL-cholesterol which is a progressive factor of hyperlipidemia and/or lowered lecithin-cholesterol acyltransferase (LCAT) so as to prevent from progression of hyperlipidemia to arteriosclerotic disease by removing stagnation of metabolism.

However, a pharmaceutical agent for improving lipid metabolism which improves lipid metabolism pertinently is not yet developed. As a therapeutic agent for treatment of abnormal lipid metabolism which is used clinically at present, Pravastatin sodium salt which inhibits 3-hydroxy-3-methyl-glutaryl-CoA reducing enzyme or clofibrate having clarification action on serum lipid is exemplified for hyperlipidemia and alimentation including high calory infusion is exemplified for hypolipidemia.
Various therapeutic agents are used depending on symptoms regardless of primary disease. Thus, there has not been any pharmaceutical agent for improving and/or treating abnormal lipid metabolism caused by imbalance of endocrinological dysfunction associated with lipid and carbohydrate metabolism. As the results of various causes, diabetes shows pathological features which are induced by imbalance of insulin and glucagon and endocrinological dysfunction-including pancreatic dysfunction, leading to metabolic disorder of liver, lipid, muscle, skin, kidney and so on. A role of adipose tissue is to stock as lipid in the case of excess of energy and to decompose lipid and supply to living body as an energy source if required. In the case of diabetes, synthesis is inhibited in adipose tissue and decomposition is stimulated because of insulin deficiency and excessive glucagon. That is, by decrease of insulin action, sucrose intake decrease in adipose tissue,resulting in decrease of production of α-glycerophosphoric acid, NADH and NADPH and decrease of synthesis of fatty acid and reesterification. On the other hand, insulin deficiency and excessive glucagon activates hormone-sensitive lipase to decompose lipid. Mechanism of development of fatty liver is thought that impairment occurs in either process of triglyceride synthesis or synthesis and secretion of VLDL in liver.
Stimulation of fatty acid synthesis in liver, increase of fatty acid mobilization from peripheral adipose tissue to liver, decrease of ability to oxidize fatty acid and decrease of synthesis and secretion of lipoprotein in liver are thought to be examples of the above mechanism. For example, in fatty liver associated with obesity, mobilization of fatty acid from peripheral adipose tissue increase because of increase of the content of peripheral adipose tissue. Recently, attention is focused on the relation between development of fatty liver and increase of fatty acid in portal vein due to accumulation visceral lipid. In addition, acidity of acetyl CoA carboxylase which is a rate determining enzyme in de novo synthesis of fatty acid in liver is enhanced in obesity, suggesting enhancement of synthesis of fatty acid in liver. On the other hand, in obesity, hyperinsulinemia is induced to compensate increase of insulin resistance in peripheral adipose tissue.

It is known that fatty liver is accompanied with obesity with hyperinsulinemia at a high rate, comparing to obesity with normal insulin secretion. Thus, with respect to abnormal linked metabolism of carbohydrate and lipid, there has not been any pharmaceutical agent for improving lipid metabolism tactically by stimulating synthesis of serum lipoprotein carrying lipid in blood, improving mobilization thereof to lipid composition and metabolism thereof, enhancing LCAT activity associated with esterification, decreasing free cholesterol level, increasing HDL-cholesterol level, stimulating choleic excretion, decreasing serum lipid level, effluxing triglyceride accumulated in hepatic cell and improving fatty liver.
Considering these situations, the present inventors have eagerly investigated on a therapeutic agent for treatment of abnormal linked metabolism of carbohydrate and lipid and found that TCF-II specifically relates to mobilization of serum lipoprotein carrying lipid in blood to lipid composition and metabolism thereof. Inhibition of synthesis thereof, decrease of LCAT activity associated with esterification and decrease of HDL accompanied therewith are regarded as serious risk factors of adult diseases accompanied with disorder of lipid metabolism such as hyperlipidemia, arteriosclerosis and so on. The present inventors found that TCF-II significantly enhanced LCAT activity in vivo, improved hypo-high-density-lipoproteinemia, further, stimulated the mobilization by increase serum lipoprotein and normalized serum lipid level. An object of the present invention is to provide a therapeutic agent having efficacy to normalize abnormal lipid metabolism comprising TCF-II as an effective ingredient.

### Summary of the Invention

An object of the present invention is to manufacture a therapeutic agent for treatment of disorder of lipid metabolism comprising TCF-II as an effective ingredient.
Another object is to provide a therapeutic agent for treatment having an action of decreasing high level of serum lipid and normalizing hypolipidemia and having efficacy to improve fatty liver.

### Brief Description of Drawings

Figure 1 shows the method of fractionation of serum VLDL fractions in example 4.
Figure 2 shows the method of fractionation of serum HDL fraction and HDL₃ fraction in example 4.
Figure 3 shows concentration of serum triacylgrycerol in example 4.
Figure 4 shows concentration of serum ester-type cholesterol and serum free cholesterol and LCAT activity in blood in example 4.
Figure 5 shows administration protocol in example 5.
Figure 6 shows content of triacylglycerol in tissue and concentration of serum phospholipid and serum total cholesterol in example 5.
Figure 7 shows concentration of serum albumin, serum HDL-cholesterol, serum free cholesterol and serum total cholesterol in example 6.
Figure 8 shows administration protocol in example 7.
Figure 9 shows LCAT activity in example 7.
Figure 10 shows administration protocol in example 8.
Figure 11 shows concentration of serum HDL-cholesterol, serum cholesterol and serum total cholesterol.

### Detailed Description of the Invention and Preferred Embodiments

A therapeutic agent for treatment of disorder of lipid metabolism comprising TCF-II as an effective ingredient of the present invention is useful for a therapeutic agent for treatment of disorder of lipid metabolism caused by diabetes, endocrinological diseases, such as thyroid dysfunction and adrenal dysfunction, chronic metabolic disorder, chronic renal failure including nephroses, uremia, cancer, dyscrasia, adverted effect of antitumor agent, gastrointestinal (including hepatic and pancreasic) impairment and so on. As a disorder of lipid metabolism described above, hypercholesterolemia, hypolipidemia or hypo-high-density-lipoproteinemia can be exemplified.

TCF-II as an effective ingredient of the present invention is a known protein derived from human fibroblast and characteristics thereof is shown as follows:
i) Molecular weight (SDS electrophoresis)
   under non-reducing conditions: 78,000±2,000, or 74,000±2,000
   under reducing conditions : 52,000±2,000 (common band)
      30,000±2,000 (band B)
      26,000±2,000 (band C)
ii) Isoelctric point : 7.4-8.6
iii)Length of amino acid : 723

TCF-II described above can be obtained by concentrating culture broth of human fibroblast, adsorbing it on ion exchanger and purifying the eluted substance by affinity chromatography (WO90/10651) or by gene manipulation (WO92/01053).

As TCF-II of an effective ingredient of the present invention, TCF-II derived from human fibroblast or TCF-II produced by gene manipulation based on gene sequence described in patent WO90/10651 using microbial or other cell can be used. In addition, TCF-II obtained by gene manipulation described in patent WO92/01053 can be also used. TCF-II with different carbohydrate chain or without carbohydrate chain due to the difference of host cell or microbial organism can be also used in the above case.

However, TCF-II with carbohydrate chain can be preferably used because carbohydrate chain relates to metabolic rate in a living body. TCF-II obtained by these method can be concentrated and purified by usual methods of isolation and purification. For example, precipitation method using organic solvent, salting-out, gel filtration, affinity chromatography using monoclonal antibody and electrophoresis etc. are exemplified. Purification by affinity chromatography using monoclonal antibody can be carried out by using monoclonal antibody described in Japanese published unexamined patent application 97(1993). Obtained TCF-II can be freeze dried or preserved in a freezer. A therapeutic agent for treatment of disorder of lipid and carbohydrate linked metabolism can be administered intravenously, intramuscularly or subcutaneously as injections. These pharmaceutical preparations can be produced according to known pharmaceutical preparation method. Controlling agent of pH, buffer, stabilizer and so on can be added thereto, if necessary. Administration dosage of the pharmaceutical preparation of the present invention in a patient can be varied depending on symptom, health condition, age or body weight etc. of a patient. As purified TCF-II, not limiting specifically, 0.6mg-600mg/ adult man/day can be administered. Preferably, a pharmaceutical preparation comprising 6mg-60mg of TCF-II can be administered once or more per day.

The present invention will be described in detail by exemplifying examples but the scope of the present invention is not limited to these examples.

### [Example 1]

### Purification of TCF-II

According to the method described in patent WO90/10651 and the method by Higashio et.al. (Higashio, K. et. al., B.B.R.C., vol.170, pp 397-404 (1990)), purified TCF-II was obtained by cell culture. Human fibroblast cells IMR-90 (ATCC CCL 186) 3x10⁶ were inoculated into a roller bottle including 100ml DMEM medium with 5% calf serum and cultured by rolling it at rotation rate of 0.5-2 times/min. for 7 days. When total cell number became 1x10⁷, cells were collected on the bottom surface by trypsin treatment, 100g of 5-9 mesh sterilized ceramics were added thereto and stationary culture thereof was kept for 24 hours. Then, 500 ml of the above culture broth was added thereto and culture was continued. The whole culture medium was collected every 7-10 days and fresh medium was supplemented. Thus, production was kept for 2 months and 4 L of culture broth per one bottle was collected. Specific activity of the culture broth obtained as described above was 32 µ/ml.

The culture broth (750 L) was concentrated by ultrafiltration using a membrane filter(MW 6,000 cut;Amicon) and purification by serial column chromatography using CM-Sephadex C-50 (Pharmacia), ConA-Sepharose (Pharmacia), Mono S column (Pharmacia) and Heparin-Sepharose (Pharmacia) was carried out to give purified TCF-II.

### [Example 2]

### Production of Recombinant TCF-II

According to the method described in patent publication WO92/01053, cells transformed with TCF-II gene were cultured and purified TCF-II was obtained. Transformed Namalwa cells were cultured and 20 L of culture broth was obtained. This culture broth was purified by CM-Sephade x C-50 (Pharmacia) chromatography, ConA-Sepharose CL-6B (Pharmacia) chromatography and HPLC packed with Mono S(Pharmacia) and about 11 mg of active TCF-II was obtained.

### [Example 3]

### Production of Pharmaceutical Preparations

Production examples of injections of recombinant TCF-II obtained in the above example 2 are shown below.

| | | |
|---|---|---|
| 1 | TCF-II | 20 µg |
| | human serum albumin | 100 mg |

The above composition was dissolved in 0.01M phosphate buffer solution with pH of 7.0 and the whole volume thereof was made up to 20 ml. After sterilization, the sample was divided into a vial 2ml each and freeze dried ,followed by sealing thereof.

| | | |
|---|---|---|
| 2 | TCF-II | 40 µg |
| | Tween 80® | 1 mg |
| | human serum albumin | 100 mg |

The above composition was dissolved in a physiological saline solution for injections and the whole volume thereof was made up to 20 ml.After sterilization, the sample was divided into a vial 2ml each and freeze dried , followed by sealing thereof.

| | | |
|---|---|---|
| 3 | TCF-II | 20 µg |
| | Tween 80® | 2 mg |
| | sorbitol | 4 g |

The above composition was dissolved in 0.01M PBS with pH of 7.0 and the whole volume thereof was made up to 20ml. After sterilization, the sample was divided into a vial 2ml each and freeze dried, followed by sealing thereof.

| | | |
|---|---|---|
| 4 | TCF-II | 40 µg |
| | Tween 80 ® | 1 mg |
| | glycine | 2 g |

The above composition was dissolved in a physiological saline solution for injections and the whole volume thereof was made up to 20 ml. After sterilization, the sample was divided into a vial 2 ml each and freeze dried, followed by sealing thereof.

| | | |
|---|---|---|
| 5 | TCF-II | 40 µg |
| | Tween 80® | 1 mg |
| | sorbitol | 2 g |
| | glycine | 1 g |

The above composition was dissolved in a physiological saline solution for injections and the whole volume thereof was made up to 20ml.After sterilization, the sample was divided into a vial 2ml each and freeze dried, followed by sealing thereof.

| | | |
|---|---|---|
| 6 | TCF-II | 20 µg |
| | sorbitol | 4 g |
| | human serum albumin | 50 mg |

The above composition was dissolved in 0.01M PBS with pH of 7.0 and the whole volume thereof was made up to 20 ml. After sterilization,the sample was divided into a vial 2ml each and freeze dried,followed by sealing thereof.

| | | |
|---|---|---|
| 7 | TCF-II | 40 µg |
| | glycine | 2 g |
| | human serum albumin | 50 mg |

The above composition was dissolved in a physiological saline solution for injections and the whole volume thereof was made up to 20ml. After sterilization, the sample was divided into a vial 2ml each and freeze dried, followed by sealing thereof.

| | | |
|---|---|---|
| 8 | TCF-II | 40 µg |
| | human serum albumin | 50 mg |

The above composition was dissolved in 0.01M PBS and the whole volume thereof was made up to 20 ml. After sterilization, the sample was divided into a vial 2 ml each and freeze dried, followed by sealing thereof.

### [Example 4]

### Stimulation of lipoprotein secretion and plasma LCAT activity in Normal Rats by TCF-II

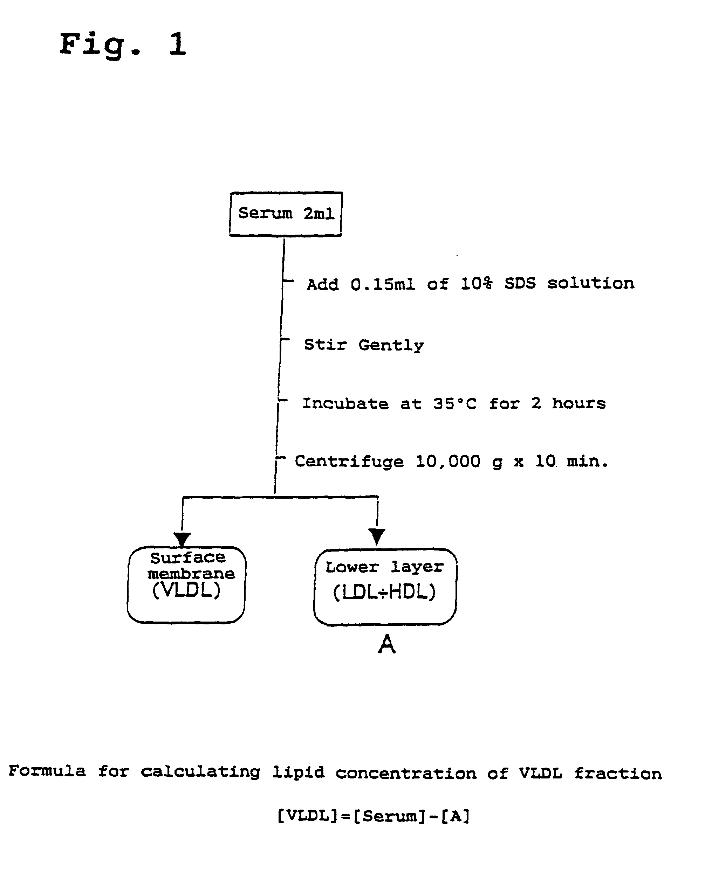

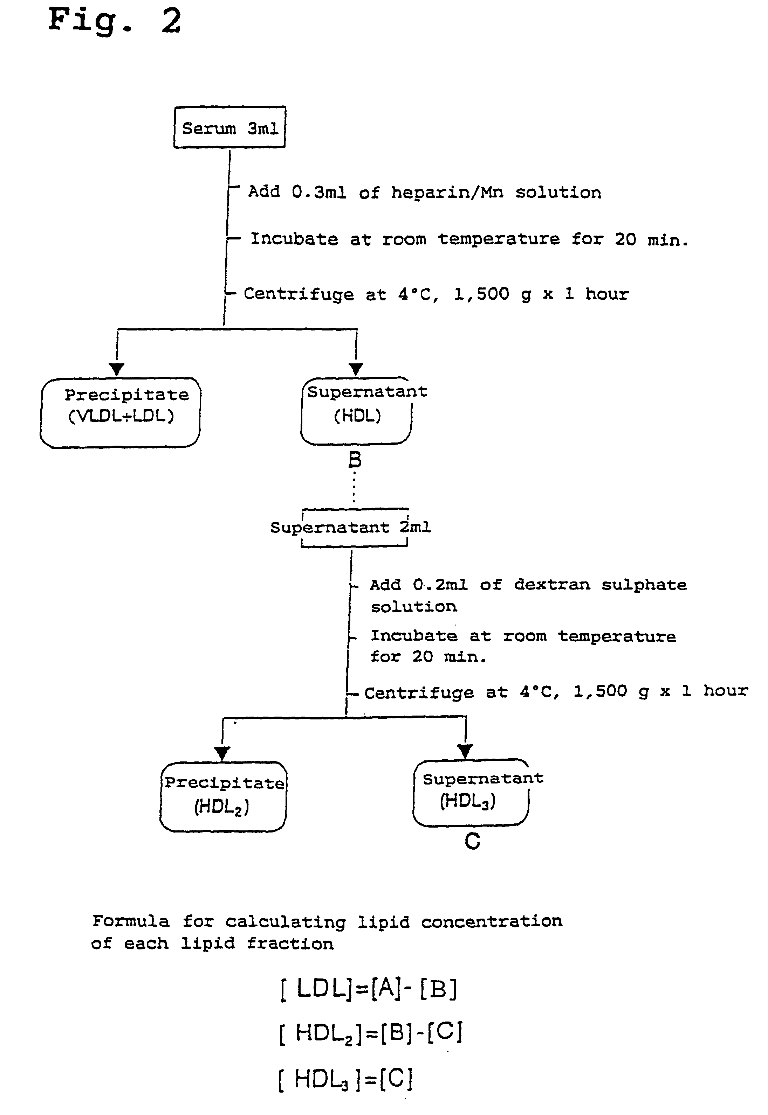

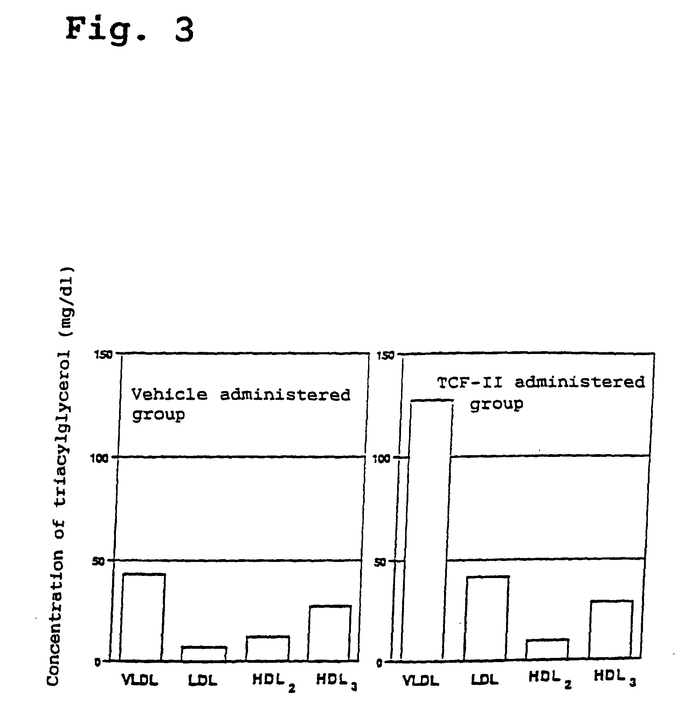

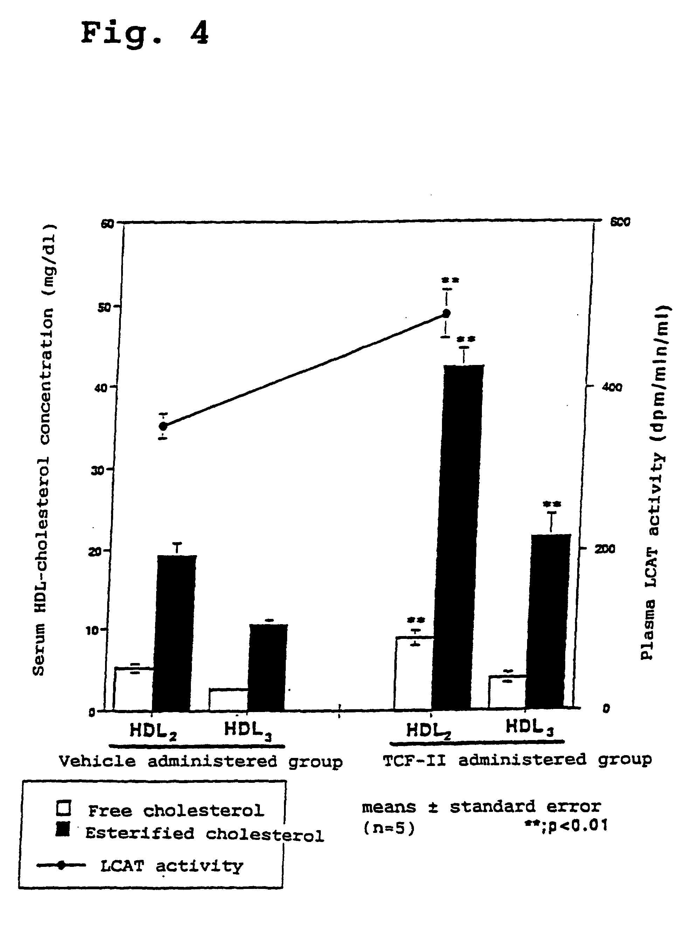

Male wistar rats (8 weeks old, 300g, 5 rats/group) were intravenously injected twice a day with 500 µg/kg of TCF-II (1000 µg/kg/day) for 4 days. Rats of control group were injected with only vehicle. Twelve hours after the last injection, blood was taken from posterior cava under ether anesthesia. Sera and plasma were collected for following analysis of lipid in lipoprotein and LCAT activity, respectively. For analysis of lipoprotein, VLDL was separated according to the methods of Wilson et. al. (Clin. Chem., vol.20, 394-395, 1974), and HDL₂ and HDL₃ were separated according to the methods of Gidez et. al. (J. Lipid Res., vol.23, 1206-1223, 1982). Schematic representations of the above procedures are illustrated in figure 1 and 2. The concentrations of triacylglycerol, total cholesterol and free cholesterol were determined enzymatically with the commercially available kits (Wako Pure Chemical Industries, Osaka, Japan). The concentration of esterified cholesterol was calculated according to the following relationship, ([concentration of total cholesterol]- [concentration of free cholesterol]}. Plasma LCAT activity was determined according to the methods of Glomset-Wright (Methods in Enzymology, vol.15, 543, 1969).
The concentration of triacylglycerol in VLDL was significantly higher in TCF-II-injected rats than in control rats (Figure 3). This result suggested that TCF-II stimulates secretion of VLDL and release of triacylglycerol into blood from liver tissue. In addition, plasma LCAT activity was significantly enhanced and the concentration of HDL cholesterol, especially esterified cholesterol, markedly increased in TCF-II-injected rats (Figure 4), which showed that TCF-II stimulated secretion of LCAT from liver so as to produce HDL.

### [Example 5]

### Reduction of Highly Accumulated Liver Triacylglycerol in Alcohol-induced Fatty Liver Rats by TCF-II

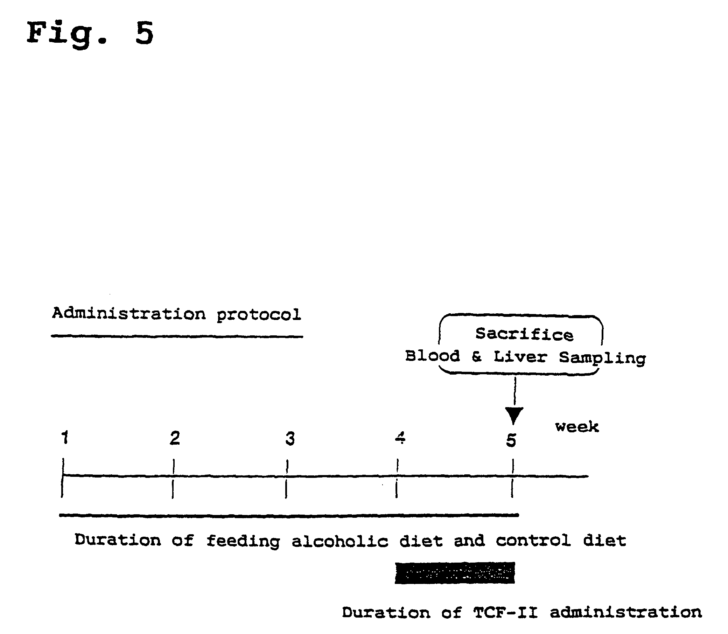

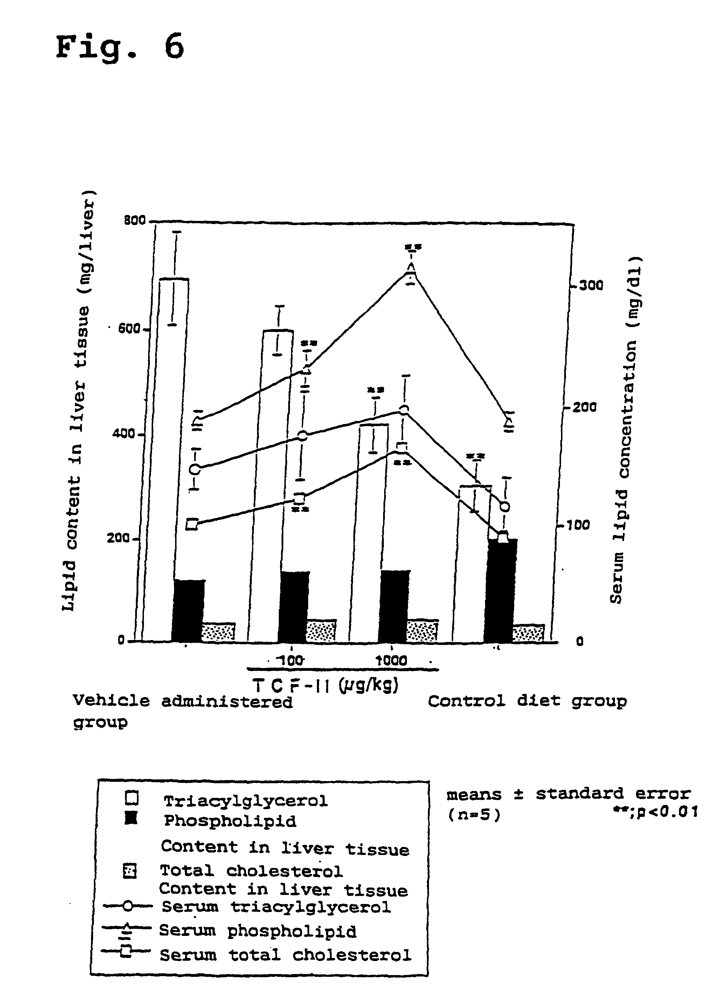

Liquid diet was prepared according to the method of Liber, et al (Trans. Assoc. Am. Physician. vol.76, 289-301, 1963). Male wistar rats (7 weeks old, 280g, 10-13 rats/group) were fed with the liquid diet containing 5% alcohol for 5 weeks and induced to fatty liver. Control rats were fed with the liquid control diet of equal calories. Fatty liver rats were intravenously injected twice a day with 50 and 500 µg/kg of TCF-II (100 and 1000 µg/kg/day, respectively) for the last 7 days of alcohol feedings. Rats of control group were injected with only vehicle.
Twelve hours after the last TCF-II injection, the animals were sacrificed and livers were excised. The liver lipids were extracted by 30 volumes of chloroform and methanol (2:1), and the contents of triacylglycerol, phospholipid and total cholesterol were determined with the commercially available kits (Wako Pure Chemical Industries, Osaka, Japan ). The administration protocol is shown in figure 5.
The highly accumulated liver triacylglycerol in fatty liver rats were dose-dependently diminished by TCF-II injection. The liver phospholipid and cholesterol levels, however, remained unchanged (Figure 6). These results also suggested that TCF-II increases triacylglycerol secretion from liver and improves fatty liver.

### [Example 6]

### Reduction of High Serum Cholesterol Concentration in α-naphthylisothiocyanate (ANIT)-induced Cholestasis Rats by TCF-II

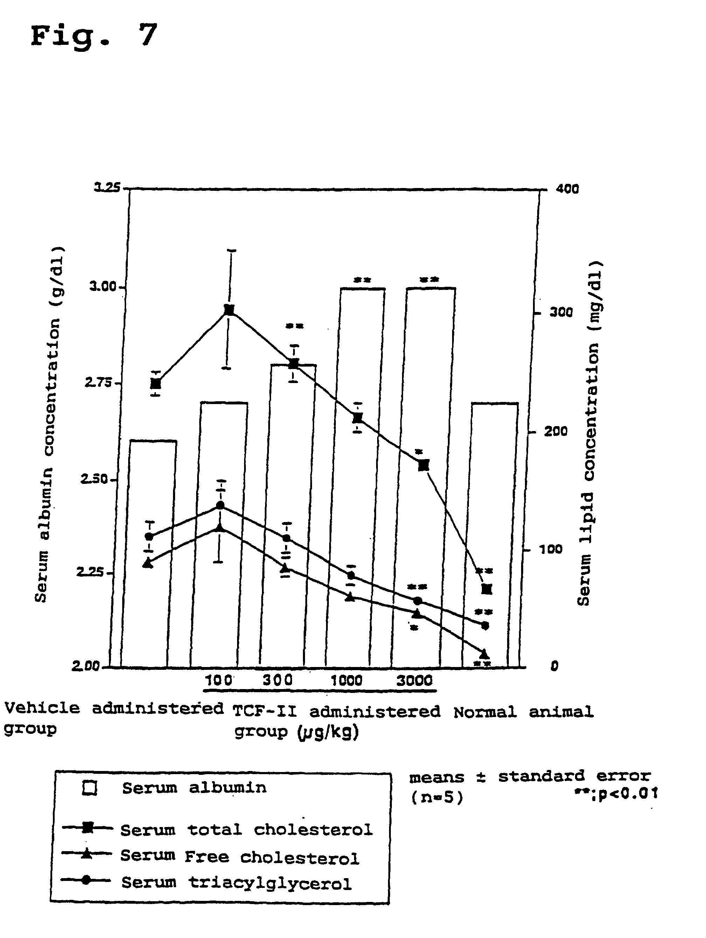

Male wistar rats (7 weeks old, 280g, 10 rats/group) were orally administered 100 mg/kg of ANIT for 5 weeks and induced to cholestasis. Cholestasis rats were intravenously injected twice a day with 50, 150, 500 and 1500 µg/kg of TCF-II (100, 300, 1000 and 3000 µg/kg/day, respectively) for 3 days immediately after administration of ANIT. Twelve hours after the last injection, blood was taken from posterior cava under ether anesthesia. Sera were collected for following measurement of serum lipid and albumin concentration. The serum concentrations of triacylglycerol, free cholesterol, total cholesterol and albumin were determined by Hi tachi 7150 Autoanalyzer.
The serum concentrations of triacylglycerol, free cholesterol, total cholesterol of the cholestasis rats was significantly higher than those of normal rats. TCF-II injection reduced the serum lipid concentrations and increased the serum albumin concentration in a dose-dependent manner (Figure 7). From these results, TCF-II improves hyperlipidemia accompanied by cholestasis.

### [Example 7]

### Stimulation of plasma LCAT activity in Dogs by TCF-II

Female and male Beagles (1-5 years old, about 10 kg, 5 dogs/group) were intravenously injected twice a day with 50, 150 and 500 µg/kg of TCF-II (100, 300 and 1000 µg/kg/day, respectively) for 7 days.

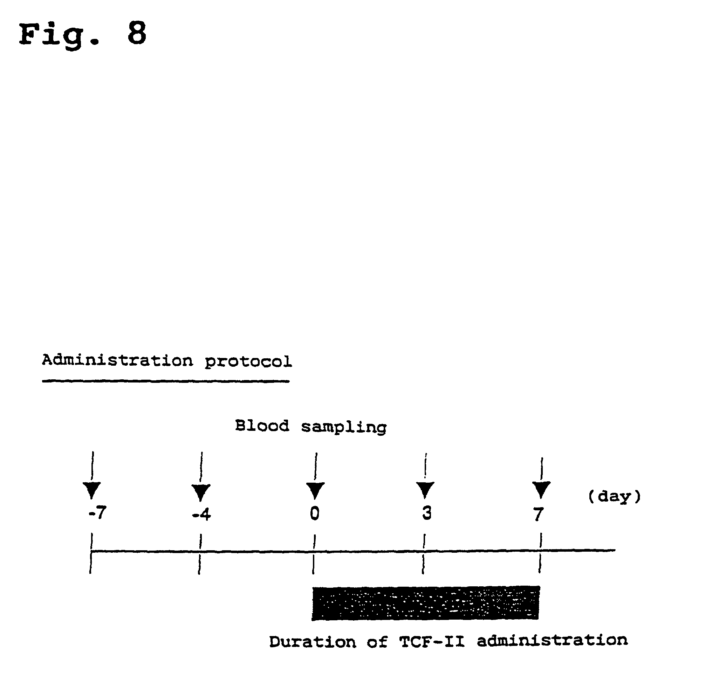

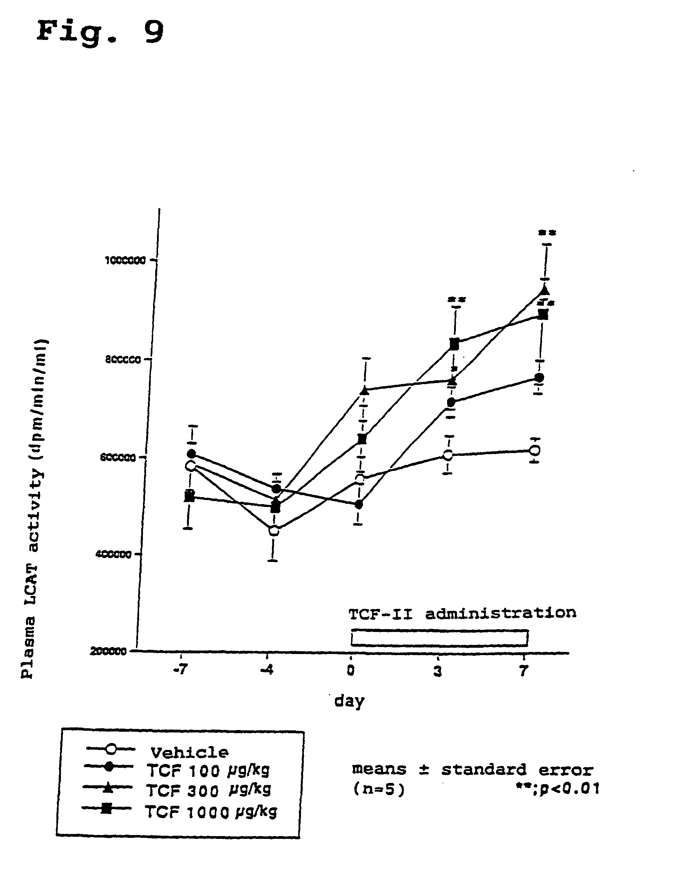

Dogs of control group were injected with only vehicle. Before and during TCF-II injection, blood was taken from forelimb vein and plasma were collected for following analysis of LCAT activity. The administration protocol is shown in figure 8.
Plasma LCAT activity was determined according to the methods of Glomset-Wright (Methods in Enzymology, vol.15, 543, 1969). Plasma LCAT activities of the TCF-II injected animals markedly increased (Figure 9), which showed that TCF-II stimulates secretion of LCAT from liver and lipid metabolism in normal dogs.

### [Example 8]

Elevation of Low Serum Cholesterol, Glucose Concentration and Low Liver Glycogen contents in Dimethylnitrosamine (DEM)-induced Cirrhosis Rats by TCF-II

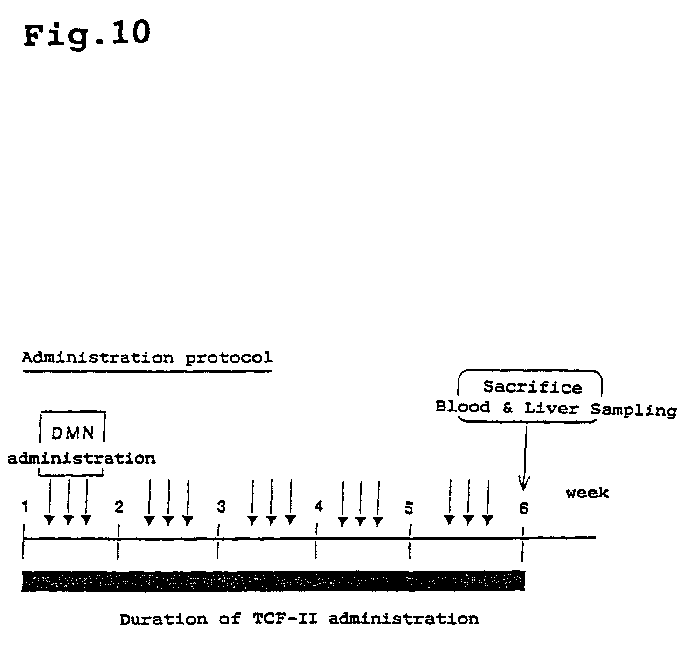

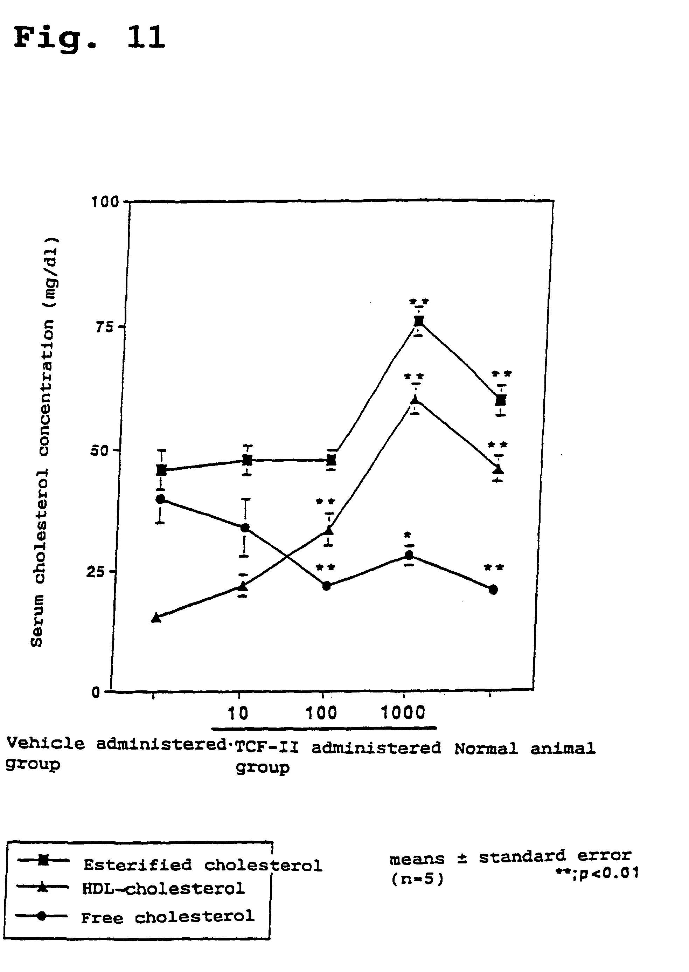

Male wistar rats (8 weeks old, 300g, 8-10 rats/group) were intraperitoneally administered 3 times a week 10 µg/kg of DEM for 4 weeks and induced to cirrhosis. Cirrhosis rats were intravenously injected twice a day with 5, 50 and 100 µg/kg of TCF-II (10, 100 and 1000 µg/kg/day, respectively) for 4 weeks during administration of DEM. The administration protocol is shown in figure 10. Twelve hours after the last injection, blood was taken from posterior cava under ether anesthesia. Sera were collected for following measurement of serum cholesterol concentration. The serum concentrations of HDL-cholesterol, free cholesterol and total cholesterol were determined by Hitachi 7150 Autoanalyzer. The serum concentrations of esterified cholesterol were calculated according to the relationship described in Example 4.
The serum concentrations of HDL-cholesterol and esterified cholesterol of the cirrhosis rats were significantly lower than those of normal rats, but the concentration of free cholesterol higher. TCF-II injection elevated the serum HDL-cholesterol and esterified cholesterol concentrations and reduced the serum free cholesterol concentration significantly (Figure 11). From these results, TCF-II improves hypo-high-density-lipoproteinemia accompanied by cirrhosis.

### Industrial Applicability

The present invention is to provide a therapeutic agent for treatment of disorder of lipid metabolism comprising TCF-II as an effective ingredient. The therapeutic agent of the present invention normalizes hyperlipidemia and hypolipidemia. It also improves fatty liver.

## Claims

1. Use of TCF-II for the preparation of a therapeutic agent for the treatment of hypercholesterolemia.

2. Use of TCF-II for the preparation of a therapeutic agent for improving lowered lecithin-cholesterol acyl transferase to prevent progression of hypo-high density-lipoproteinemia.

## Patentansprüche

1. Verwendung von TCF-II für die Herstellung eines therapeutischen Mittels für die Behandlung von Hypercholesterinämie.

2. Verwendung von TCF-II für die Herstellung eines therapeutischen Mittels zum Verbessern gesenkter Lecithin-Cholesterin-Acyl-Transferase, um ein Fortschreiten einer Hypolipoproteinämie von Lipoproteinen hoher Dichte (HDL - Hypolipoproteinämie) zu verhindern.

## Revendications

1. Utilisation du TCF-II pour la préparation d'un agent thérapeutique pour le traitement de l'hypercholestérolémie.

2. Utilisation du TCF-II pour la préparation d'un agent thérapeutique pour améliorer la lécithine - cholestérol acyltransférase diminuée et ainsi prévenir la progression de la diminution du taux sanguin des lipoprotéines de haute densité.
